# EUROPEAN PATENT APPLICATION

(11) **EP 4 663 126 A1**
(43) Date of publication of application: **17.12.2025**
(21) Application number: 24305924.3
(22) Date of filing: 12.06.2024
(51) Int. Cl.: A61B 5/378

(54) **METHOD FOR OBJECTIVELY ASSESSING A LEVEL OF VISUAL SENSITIVITY TO LIGHT OF A SUBJECT, CORRESPONDING DEVICE AND EYEWEAR**

(71) Applicant: Essilor International, 94220 Charenton-Le-Pont (FR)
(72) Inventor: TARTAGLIA, Elisa, 75011 PARIS (FR); SALVATI, Valerio, 75012 PARIS (FR)
(74) Representative: Jacobacci Coralis Harle

(57) **Abstract**

Method for objectively assessing a level of sensitivity to a light entering at least one eye of a subject, comprising steps: a) measuring and recording a cortical activity signal of a cortex of a head of the subject while the at least one eye of the subject receives a given light intensity during a given measuring and recording time, b) processing the recorded cortical activity signal to obtain a useful signal and to identify, from the useful signal, a specific cortical signature indicative of a sensitivity to light, c) assessing whether the specific cortical signature correlates with a hypo-sensitivity to light or a hyper-sensitivity to light state of the subject by comparing the specific cortical signature to a predefined threshold parameter of the specific cortical signature, wherein the given light intensity is less or equal to 50 lux and that the given measuring and recording time is less than or equal to 350 ms.

## Description

### TECHNICAL FIELD OF THE INVENTION

The domain of the invention delves into the realm of light sensitivity. The invention relates to a method for objectively assessing a level of visual sensitivity to light of a subject and also relates to a corresponding device and eyewear. It has applications in the fields of ophthalmology and optometry.

### BACKGROUND INFORMATION AND PRIOR ART

The physiological and neurological responses of the visual system of human beings to light exposure, notably as regards the light sensitivity, have been explored and is now better understood.

This domain of light sensitivity encompasses the exploration and understanding of the human body's physiological and neurological responses to various light intensities and conditions.

It is not merely about recognizing the visual discomfort some individuals might experience upon exposure to specific luminous stimuli but delving deeper into the very fabric of how the human brain perceives, processes, and reacts to such stimuli.

Light sensitivity as a subject of study is multifaceted. It interlinks the fields of neuroscience, ophthalmology, and even psychology, offering a holistic approach to comprehending the myriad ways individuals can be affected by light. The spectrum of light sensitivity can range from mild visual discomfort in bright conditions, often manifested as a mere squint, to severe pain or headaches triggered by even minimal light exposure.

Furthermore, the implications of this field are vast. Beyond the immediate physiological responses, understanding visual light sensitivity can lead to innovations in lighting design, advancements in therapeutic interventions for conditions like migraines, and the development of protective eyewear tailored to individual needs.

In today's medical and technological landscape, the evaluation of light sensitivity, predominantly relies on subjective methodologies. These subjective evaluations, although widely employed, come with their own set of challenges and limitations, chief among them being the inherent variability of human perception and the difficulty of standardizing such assessments.

Historically, determining an individual's visual sensitivity to light has necessitated direct feedback from the individual. This feedback often encompasses descriptions of discomfort, pain, or other sensations experienced upon exposure to light of varying intensities and conditions. While this approach does provide valuable insights into an individual's experience, it inevitably introduces a degree of subjectivity. Every person's threshold of discomfort, and their ability to articulate it, can vary significantly. This makes it challenging to establish a universally applicable standard or baseline for sensitivity.

Furthermore, the reliance on personal feedback has a few additional implications. For one, it becomes difficult to fully automate the evaluation process. Automation requires consistent and quantifiable data, something that subjective feedback struggles to provide. In contrast, objective measures, such as those derived from internal bodily responses, can be consistently quantified, enabling a smoother transition to automation.

Therefore, the determination of the light sensitivity is generally done with subjective experiments for assessing the discomfort or not to light and in which at least one eye of a human subject, which is subjected different light conditions, has to answer whether he/she feels a discomfort or not and the level of the discomfort.

Such subjective assessments have drawback due to the large variability of the subjective responses of the subjects, notably due to various contextual factors.

It is proposed in the invention a determination of the light sensitivity of a subject based on an objective, specifically brain-based, methodology.

The invention answers the need to distinguish objectively between subjects who are sensitive to light and those who are not, notably to be able to provide a customized ophthalmic solution for both.

Document WO2022058456A1 discloses a method for assessing objectively a glare state of a subject.

### SUMMARY OF THE INVENTION

The goal of the current invention is to provide an objective and more efficient solution thanks to a reliable and unambiguous signature of a visual sensitivity to light and a threshold of the signature delimiting between hypo-sensitivity to light and hyper-sensitivity to light, from recordings of the cortical activity of the subject.

The invention relates to a method for objectively assessing a level of sensitivity to a light entering at least one eye of a subject, comprising steps of:
a) measuring and recording a cortical activity signal of a cortex of a head of the subject while the at least one eye of the subject receives a given light intensity during a given measuring and recording time,
b) processing the recorded cortical activity signal to obtain a useful signal and to identify, from the useful signal, a specific cortical signature indicative of a sensitivity to light,
c) assessing whether the specific cortical signature correlates with a hypo-sensitivity to light or a hyper-sensitivity to light state of the subject by comparing the specific cortical signature to a predefined threshold parameter of the specific cortical signature,
wherein the given light intensity is less or equal to 50 lux and that the given measuring and recording time is less than or equal to 350 ms.

In various embodiments of the method, the following means and characteristics, which can be used alone or in any technically possible combination, are used:
- the assessment of step c) on the identified specific cortical signature is done with a classifier using the predefined threshold parameter;
- the measuring and recording of the cortical activity signal is iterated to obtain a plurality of recorded cortical activity signals that are averaged together into an averaged recorded cortical activity signal and the step b) is processing the averaged recorded cortical activity signal to obtain a useful signal and to identify the specific cortical signature;
- the averaged recorded cortical activity signal is the result of an average of at least five obtained cortical activity signals;
- the measuring and recording of the cortical activity signal and the processing of the recorded cortical activity signal to obtain a useful signal are iterated to obtain a plurality of useful signals that are averaged together into an averaged useful signal and said averaged useful signal is processed to identify the specific cortical signature indicative of a sensitivity to light that is then assessed at step c);
- the averaged useful signal is the result of an average of at least five obtained cortical activity signals;
- the useful signal that is used to identify the specific cortical signature at step b) is a result of an averaging of iterated measured and recorded cortical activity signals or of iteratively obtained useful signals;
- the number of iterations is comprised between five and twenty-five;
- the number of iterations is twenty;
- the number of iterations is five;
- on average one iteration lasts less than four seconds;
- the measuring and recording of the cortical activity signal start from the moment the subject starts receiving the given light intensity;
- said measuring and recording time is greater than 50 ms;
- said given light intensity is greater than 20 lux;
- preferably, said measuring and recording time is 300 ms;
- preferably, said given light intensity is 45 lux;
- the processing of the recorded cortical activity signal is at least one of a filtering, a filling-in and an averaging;
- the processing of the recorded cortical activity signal comprises a filtering;
- the useful signal is issued from a filtered recorded cortical activity signal;
- the useful signal issued from a filtered recorded cortical activity signal is a band limited signal between 1 Hz and 40 Hz, excluding signals outside said band;
- the filtered recorded cortical activity signal is filtered with a high-pass filter at 1 Hz and a low-pass filter at 40 Hz;
- the filtered recorded cortical activity signal is filtered with a band-pass filter from 1 Hz to 40 Hz;
- the processing of the recorded cortical activity signal comprises a filling-in;
- the recorded cortical activity signal useful signal is processed to identify, remove and replace artifacts with filling in through interpolation;
- the processing of the recorded cortical activity signal comprises an averaging of iteratively measured and recorded cortical activity signals or of iteratively obtained useful signals;
- in the iterations, the intensity of the given light intensity remains the same;
- in the iterations, the time of the given measuring and recording time remains the same;
- in the iterations, both the intensity and the time remain the same for the given light intensity and the given measuring and recording time;
- the useful signal used to identify the specific cortical signature is an average of a number of recorded cortical activity signals measured and recorded at step a), or of obtained useful signals at step b);
- the useful signal used to identify the specific cortical signature is an average of five to twenty-five recorded cortical activity signals, or of obtained useful signals at step b);
- advantageously, the useful signal used to identify the specific cortical signature is an average of twenty recorded cortical activity signals, or of obtained useful signals at step b);
- the cortical activity signal is measured by an Electroencephalographic system (EEG);
- the cortical activity signal measured by an Electroencephalographic system (EEG), is measured and recorded as channels;
- the measured and recorded channels are frontal and occipital and mid-line channels;
- the measured and recorded frontal and occipital and mid-line channels are FPz, FP1, FP2, O1, Oz, Fz, Cz, CP1, CP2, CPz, POz;
- the measured and recorded frontal and occipital and mid-line channels are : FPz, FP1, FP2, Fz, O2, Fz, Cz, CP1, CP2, CPz, POz;
- at step b), the useful signal is processed to identify a plurality of specific cortical signatures and at step c), the assessment is performed by comparing each of the specific cortical signatures to corresponding predefined threshold;
- the cortical activity signal which is measured and recorded in step a) comprises regional parts originating from a plurality of different regions of the cortex and wherein the useful signal which is used to identify the specific cortical signature in step b) is obtained from at least these regional parts of the cortical activity signal originating from different regions of the cortex;
- the cortical activity signal which is measured and recorded in step a) comprises three regional parts originating respectively from the occipital and frontal and mid-line regions of the cortex and wherein the useful signal which is used to identify the specific cortical signature in step b) is obtained mainly from these three regional parts of the cortical activity signal;
- the cortical activity signal is measured and recorded exclusively from the occipital and/or frontal regions of the cortex;
- the cortical activity signal is measured and recorded exclusively from the occipital region of the cortex;
- the cortical activity signal is measured and recorded exclusively from the frontal region of the cortex;
- the cortical activity signal is measured and recorded exclusively from the mid-line region of the cortex;
- the cortical activity signal is measured and recorded from only one cranial electrode and a reference electrode;
- the reference electrode is arranged on one ear of the subject;
- the reference electrode is arranged on the head of the subject, behind an ear of the subject;
- the reference electrode is arranged on the head of the subject, at any site on the head of the subject and that is not involved with muscle movement;
- preferably, the only one cranial electrode from which the cortical activity signal is measured and recorded is the one from a channel selected in O1, 02, Fz, Cz, CP1, CP2, CPz, POz channels;
- most preferably, the only one cranial electrode from which the cortical activity signal is measured and recorded is the one from a channel selected in O1, O2 channels;
- the signature and threshold are defined in the time domain from the amplitude of the cortical activity signal;
- the specific cortical signature is the evolution over time of an amplitude of the cortical activity signal of the cortex in the first 350 ms starting from the moment the at least one eye of the subject starts receiving the given light intensity;
- the predefined threshold parameter is a statistical value or a set of statistical values descriptive of the evolution over time of an amplitude of the cortical activity signal and comprising at least one value from mean of the amplitude, standard deviation of the amplitude, variance of the amplitude, root mean square of the amplitude, minimum amplitude equal to 0µV, negative peak maximum absolute amplitude, positive peak maximum amplitude, time latency of a determined peak, peak-to-peak time separation of determined peaks;
- the time latency of a determined peak is a delay of the positive peak maximum amplitude from 0 ms, i.e. from the starts of the recording and of the subject receiving the given light intensity;
- the peak-to-peak time separation of determined peaks is the peak-to-peak time separation from the highest positive peak to the next one positive peak over time;
- the peak-to-peak time separation of determined peaks is the peak-to-peak time separation from the highest positive peak to the highest positive peak of lower amplitude;
- steps a) and b) are iterated for a determined number of iterations on the same subject, variable blackout periods (i.e. periods with not light at all) being implemented between the iterations, wherein during each blackout period the subject receives no-light and each variable blackout period duration is randomly comprised between 2 to 5 seconds;
- the iteration of steps a) and b) is for averaging the signals,
- the determined number of iterations is comprised between five and twenty-five;
- the determined number of iterations is twenty;
- the determined number of iterations is five;
- the predefined threshold parameter of the specific cortical signature is determined in a preliminary threshold determination step comprising a statistical computation of data related to cortical activities under different levels of light intensities of a plurality of individuals from a population distinct from the subject whose level of sensitivity to light is to be assessed in relation to the feeling of each individual as to whether he feels comfortable or uncomfortable (or discomfortable, those two words being considered similar) with the light;
- the specific cortical signature is predetermined in the preliminary threshold determination step;
- the statistical computation is done with a machine learning classification process;
- the machine learning unit implements at least one of the following models: logistic regression, linear support Vector Machines (LSVM), quadratic support Vector Machines (QSVM), cubic support Vector Machines (CSVM), narrow neural networks (NNN), trilayered neural networks (TNN), medium neural networks (MNN) and wide neural networks (WNN);
- preferably, the machine learning unit implements a Support Vector Machine (SVMs),
- the predefined threshold parameter of the specific cortical signature is statistically computed from cortical activity signals and subjective responses of the individuals of the population, the cortical activity signals and subjective responses being obtained while at least one eye of each of the individuals is receiving a determined level of light intensity, for each individual multiple cortical activity signals and subjective responses being obtained for multiple levels of light intensity, the subjective response being whether the individual fells comfortable or uncomfortable to a current level of light intensity, the cortical activity signals being processed to identify specific cortical signatures indicative of a sensitivity to light, and the predefined threshold parameter of the specific cortical signature is computed as being characteristic of a statistical boundary on the specific cortical signatures between two statistically defined sub-populations from the defined population, a first sub-population of subjectively hypo-sensitive individuals and a second sub-population of subjectively hyper-sensitive individuals;
- an individual is subjectively hyper-sensitive if the individual fells uncomfortable for light intensities between 1 and 1000 lux;
- an individual is subjectively hypo-sensitive if the individual fells comfortable for light intensities between 2000 and 12,000 lux;
- an individual is subjectively hyper-sensitive if the individual fells uncomfortable for an averaged light intensities between 1 and 1000 lux;
- an individual is subjectively hypo-sensitive if the individual fells comfortable for an averaged light intensities between 2000 and 12,000 lux;
- an averaged light intensity is the average of two light intensities giving the individual bearable uncomfortable feeling and then unbearable uncomfortable feeling from two successive increased light intensities to which the individual is subjected;
- the preliminary threshold determination step comprises the sub-step of, for each individual:
   -- measuring and recording a plurality of cortical activity signals while the at least one eye of the individual receives each time a different level of light intensity during a measuring and recording time less than or equal to 350 ms and processing the recorded cortical activity signals to obtain useful signals,
   -- for each of the levels of light intensities, recording the feeling of the individual as to whether he feels comfortable or uncomfortable, and
- for all the individuals:
   -- comparing with each other the useful signals and feelings of each individual or of all the individuals of the population to statistical compute said predefined threshold parameter of the specific cortical signature for which a change occurs between comfortable feelings and uncomfortable feelings;
- in the preliminary threshold determination step the different levels of light intensities are selected between 45 and 12,000 Lux;
- the predefined threshold parameter of the specific cortical signature is determined in a preliminary threshold determination step comprising a statistical computation of data related to cortical activities under different levels of light intensities of the subject which level of sensitivity to light is to be assessed, in relation to the feeling of the subject as to whether he/she feels comfortable or uncomfortable with the light;
- the preliminary threshold determination step comprises the sub-step of:
   -- measuring and recording a plurality of cortical activity signals of said subject while the at least one eye of the subject receives each time a different level of light intensity during a measuring and recording time less than or equal to 350 ms and processing the recorded cortical activity signals to obtain useful signals,
   -- for each of the light intensities, recording the feeling of the subject as to whether he/she feels comfortable or uncomfortable,
   -- comparing with each other the useful signals and feelings of the subject to statistically compute said predefined threshold parameter of the specific cortical signature for which a change occurs between comfortable feelings and uncomfortable feelings;
- the subject is subjectively hypo-sensitive if he/she fells comfortable for light intensities between 2000 and 12,000 lux;
- the subject is subjectively hyper-sensitive if he/she fells uncomfortable for light intensities between 1 and 1000 lux;
- the subject is subjectively hyper-sensitive if he/she fells uncomfortable for averaged light intensities between 1 and 1000 lux;
- the subject is subjectively hypo-sensitive if he/she fells comfortable for averaged light intensities between 2000 and 12,000 lux;
- an averaged light intensity is the average of two light intensities giving the subject bearable uncomfortable feeling and then unbearable uncomfortable feeling from two successive increased light intensities to which the subject is subjected;
- in the preliminary threshold determination step, the measuring and recording of the cortical activity signal for each level of light intensity is iterated to obtain a plurality of recorded cortical activity signals that are averaged together into an averaged recorded cortical activity signal that is then processed;
- in the preliminary threshold determination step, the averaged recorded cortical activity signal is the result of an average of at least five obtained cortical activity signals;
- in the preliminary threshold determination step, the measuring and recording of the cortical activity signal and the processing of the recorded cortical activity signal to obtain a useful signal are iterated to obtain a plurality of useful signals that are averaged together into an averaged useful signal that is then used for the comparison;
- in the preliminary threshold determination step, the averaged useful signal is the result of an average of at least five obtained and processed cortical activity signals;
- in the preliminary threshold determination step, the number of iterations at each level of light intensity is comprised between five and twenty-five;
- in the preliminary threshold determination step, the number of iterations at each level of light intensity is twenty;
- in the preliminary threshold determination step, the number of iterations at each level of light intensity is five;
- the method further comprises a step of determining, based on the result of assessing step c), a parameter that is characteristic of a light filter to be provided to the subject in order to maintain or improve the visual comfort and/or visual acuity of the subject.

The invention also relates to a device comprising means specifically configured for the execution of the method of the invention.

More precisely, the device for objectively assessing a level of sensitivity to a light entering at least one eye of a subject comprises a control unit configured to execute the steps of:
a) measuring and recording a cortical activity signal of a cortex of a head of the subject while the at least one eye of the subject receives a given light intensity during a given measuring and recording time, the given light intensity being less or equal to 50 lux and the given measuring and recording time is less than or equal to 350 ms,
b) processing the recorded cortical activity signal to obtain a useful signal and to identify, from the useful signal, a specific cortical signature indicative of a sensitivity to light,
c) assessing whether the specific cortical signature correlates with a hypo-sensitivity to light or a hyper-sensitivity to light state of the subject by comparing the specific cortical signature to a predefined threshold parameter of the specific cortical signature,
said device comprising at least one light source to provide the given light intensity during the given measuring and recording time.

In various embodiments of the device, the means used for the method and/or the following means and characteristics, which can be used alone or in any technically possible combination, are used:
- the given light intensity is a value of light intensity that is not uncomfortable to the subject;
- the given light intensity is a value of light intensity that is comfortable to the subject;
- the useful signal that is used to identify the specific cortical signature at step b) is a result of an averaging of iterated measured and recorded cortical activity signals or of iteratively obtained useful signals;
- the number of iterations is comprised between five and twenty-five;
- the number of iterations is twenty;
- the number of iterations is five;
- the device comprised at least one cranial electrode.

The invention also relates to an eyewear comprising means specifically configured for the execution of the method of the invention.

More precisely, the eyewear comprises:
- a device for objectively assessing a level of sensitivity to a light entering at least one eye of a subject and comprising at least one cranial electrode and a control unit configured to execute the steps of:
   a) measuring and recording a cortical activity signal of a cortex of a head of the subject while the at least one eye of the subject receives a given light intensity during a given measuring and recording time, the given light intensity being less or equal to 50 lux and the given measuring and recording time is less than or equal to 350 ms,
   b) processing the recorded cortical activity signal to obtain a useful signal and to identify, from the useful signal, a specific cortical signature indicative of a sensitivity to light,
   c) assessing whether the specific cortical signature correlates with a hypo-sensitivity to light or a hyper-sensitivity to light state of the subject by comparing the specific cortical signature to a predefined threshold parameter of the specific cortical signature.

In various embodiments of the eyewear, the means used for the method and/or the following means and characteristics, which can be used alone or in any technically possible combination, are used:
- the given light intensity is a value of light intensity that is not uncomfortable to the subject;
- the given light intensity is a value of light intensity that is comfortable to the subject;
- the device comprised at least one cranial electrode;
- the eyewear comprises a light source to provide the subject with the given light intensity ;
- the useful signal that is used to identify the specific cortical signature at step b) is a result of an averaging of iterated measured and recorded cortical activity signals or of iteratively obtained useful signals;
- the number of iterations is comprised between five and twenty-five;
- the number of iterations is twenty;
- the number of iterations is five;
- the eyewear comprises a head-mounted light source isolating the eyes of the subject (or of the individual) from environmental illumination and providing the subject with the given light intensity.

Thanks to the invention, the assessment of the level of sensitivity to light entering the eye(s) of a subject is rapid, typically lasting 1 minute and 20 seconds. It is entirely not invasive as it can be implemented with only one cranial electrode on the scalp. It is highly accurate, 95% accuracy at minimum. Moreover, it exposes the subject to a single, low intensity level of light, which is chosen not to yield any discomfort even to very sensitive subjects. Finally, the assessment is completely objective because it considers the subject's internal states, i.e. the cortex activity, and not only the reported subjective feelings.

The invention can have many applications: a subject classified as hyper-sensitive can prevent disorders due to light exposure by intervening with targeted solutions such as prescribing the best ophthalmic lenses for the subject. The proposed method is also helpful in determining whether there may be medical problems to be investigated, given the subject's extreme sensitivity or non-sensitivity. Moreover, the method can be further developed into a Brain-Computer-Interface system comprising an ophthalmic device, which can modulate in real time some components, notably lenses or filters of the ophthalmic device, according to the subject internal, objective, state of sensitivity.

### List of figures

Figure 1 is a schematic view of an embodiment of a device designed to objectively detect in-real-time a subject's response to light regarding the visual sensitivity to light and that features lenses that can dynamically change colours or transmission index or employ filters and that also features at least one cranial electrode to measure a cortical activity signal of the subject wearing the device,
Figure 2 is a flowchart of the method of the invention,
Figure 3 is a representation of the light intensity increasement during the statistical study for the predetermination of the specific cortical signature and of the predefined threshold parameter of said specific cortical signature,
Figure 4 is a schematic representation of the response of an individual subjected to steps of increasing light intensities,
Figure 5 is a representation of obtained levels of light intensities at which individuals that are participants to a sensitivity characterization feel uncomfortable in the case of a statistical study with 22 individuals, one of them being not characterizable (i.e. subject number 12 "bad"),
Figure 6 is a schematic representation of the way the individual or subject receives the given light intensity for the EEG recording.

### DETAILED DESCRIPTION OF EXAMPLE(S)

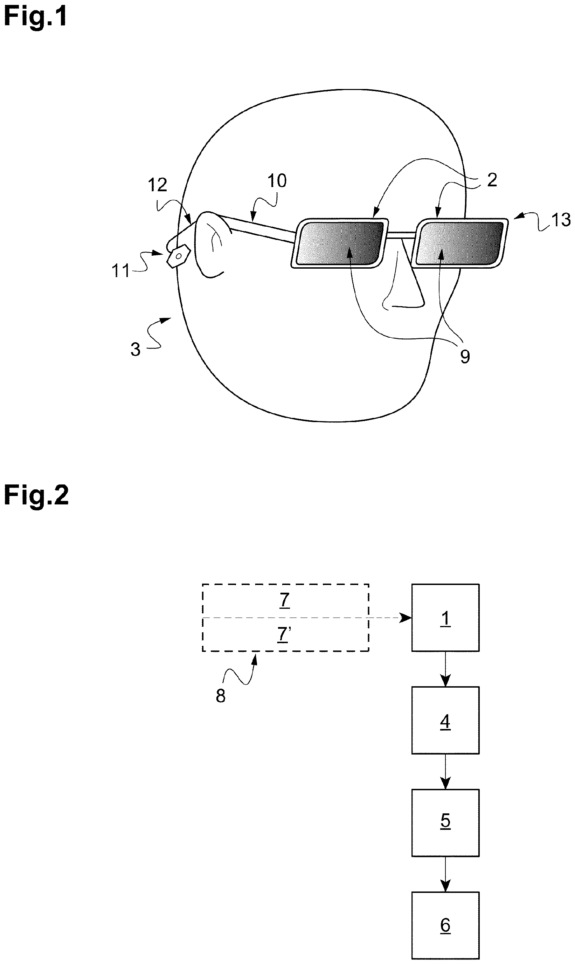

An embodiment of the device of the invention is now described in relation to figure 1. This embodiment is an eyewear 13 comprising the device for assessing a level of visual sensitivity to light of a subject. The device comprises a control unit configured to execute the steps required for the assessment. In figure 1 a head of a subject 3 is schematized. The subject 3 is wearing the eyewear 13 on his/her eyes 2. The eyewear 13 comprises active glasses 9, a frame 10 comprising electronic circuits and a power supply, e.g. a rechargeable battery, a connector 12. The connector 12 is connected to a cranial electrode 11 arranged on the scalp of the subject 3, on the occipital region. The eyewear also comprise an embedded reference electrode (not visible) typically arranged on the frame to contact one of the ears of the subject 3 or contact an area of the scalp. The electronic circuit in the frame comprises the control unit. In a simpler embodiment of the eyewear, the control unit is external and the electronic circuit of the frame comprises a communication circuit to communicate with an external device comprising the control unit for the assessment.

In this example, a single cranial electrode is placed on the back of the scalp of the head, in the occipital region, which is responsible for the processing of the visual information. This cranial electrode can be mounted on a comfortable headband worn by the subject.

In a different embodiment, three cranial electrodes can be applied to the scalp in the frontal region.

The eyewear 13 can objectively assess the level of visual sensitivity to light of the subject and it can also provide remedies in the case a high-sensitivity is assessed thanks to the active glasses 9 that can operate as filters which level of filtering can be modified by the control unit. Therefore, the assessment method can be developed into a Brain-Computer-Interface device, to pilot in real time optical lenses, e.g. E-Chromics.

The eyewear 13 of figure 1, is used with an external light source to provide the subject with the given light intensity; 45 lux in an example of implementation. The shape of external light source is typically a dome and this dome is arranged in a darkened room.

In another embodiment, the eyewear comprises the light source and is an eyewear with a head-mounted light source isolating the eyes of the subject (or of the individual or subject if the eyewear is used in the preliminary threshold determination step) from environmental illumination and providing the subject with the given light intensity.

The method to identify physiologically, objectively, and unambiguously the level of sensitivity of a subject is now described in relation to figure 2.

The step referenced 1 is a step of measuring and recording a cortical activity signal of the subject while at least one eye 2 of the subject 3 receives a given light intensity during a given measuring and recording time. The step referenced 4 is a step of processing the recorded cortical activity signal to obtain a useful signal. The step referenced 5 is a step for identifying, from the useful signal, a specific cortical signature indicative of a sensitivity to light. The step referenced 6 is a step for assessing whether the specific cortical signature correlates with a hypo-sensitivity to light or a hyper-sensitivity to light state of the subject.

In those steps, the signature for the specific cortical signature to be identified and the threshold parameter of the specific cortical signature are predefined data that have been determined previously on the subject itself or on a defined population of individuals thanks to a statistical study. This/those previous determinations are referenced 7 as a step for predetermining the threshold parameter of the specific cortical signature and referenced 7' as a step for predetermining the specific cortical signature in a preliminary threshold determination step referenced 8. Note that the step 7' for predetermining the specific cortical signature in a preliminary threshold determination step 8 can be omitted if the signature is already known. Same for the step 7 for predetermining the threshold parameter of the specific cortical signature, if the threshold is already known.

The given measuring and recording time is preferably 300ms from the moment the subject starts receiving the given light intensity. In other words, the initial 300ms of cortex response of the subject that is exposed to the given light intensity is analysed.

The given light intensity is a very dim light, preferably an intensity of 45 lux. In all cases, the given light intensity is lower than the intensity of light that gives discomfort to the subject. The specific cortical signature and the threshold parameter of the specific cortical signature that are used for the assessment can be predefined on the subject itself or on individuals from a determined population with a statistical study that can be executed in a preliminary threshold determination step on the subject or on the population respectively. Said preliminary threshold determination step is preferably done only one time, in any case before the assessment could be done if the specific cortical signature and the threshold parameter are not already known.

The statistical study of the preliminary threshold determination step that allows the identification of the specific cortical signature and the computation of the threshold parameter is now explained. It must be noted that the predefined specific cortical signature and threshold parameter for the subject itself are obtained in a similar way except the study is done on the subject itself and not on individuals from a determined population.

The statistical study is implemented according to the following phases:
- In a preliminary phase of the statistical study, a sensitivity characterization is done to determine classes of sensitivity. In this preliminary phase, the individuals of the population are exposed to varying intensities and types of light, and their levels of discomfort are recorded, typically using subjective scales. No EEG is involved in this preliminary phase of the statistical study. It is a method of understanding, at a fundamental level, how different individuals perceive, i.e. feel, and react to light stimuli. It allows the definition and the characterization of two classes of individuals: individuals that are hyper-sensitives and individuals that are hypo-sensitives to light and the corresponding levels of light intensities.

For that purpose, during a subjective assessment in the preliminary phase of the statistical study, each individual is seated on an armchair, resting their head on a chinrest. The individual is positioned facing a dome light that provides an even and homogeneous illumination at least in his/her face and eyes. This setup took place in a darkened room, with the individual's gaze fixated on the centre of the dome.

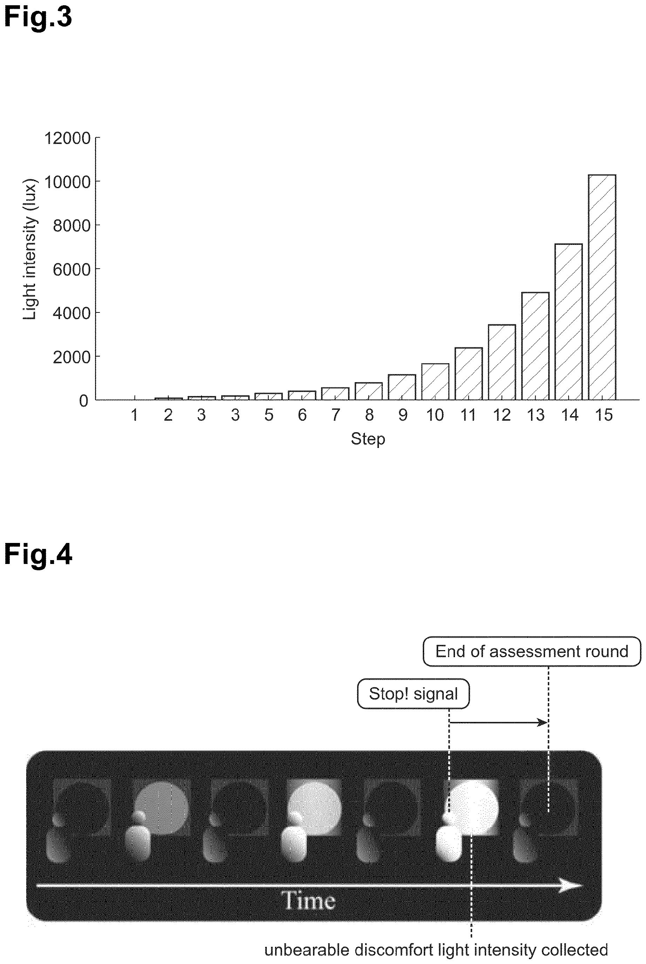

The dome projects a series of light flashes, each lasting 500ms, with exponentially increasing intensities. The increasing light intensities are represented as steps in Figure 3. In this example, 15 levels of light intensities are possible, the intensity of the light at each step being represented as lux values. Still in this example, the light intensities increased from 45 lux to 10255 lux.

These flashes are alternated with periods of darkness/blackout, lasting 2 seconds.

An example of sequences of light flashes and periods of darkness/blackout submitted to an individual is schematized in Figure 4.

Initially, individuals are instructed to press a first button when they feel the light's intensity uncomfortable (i.e. bearable discomfort) and a second button when they feel the light's intensity unbearable (i.e. unbearable discomfort).

Consequently, during the study, each individual presses the first button each time he feels a bearable discomfort and the second button when it is challenging (i.e. unbearable discomfort) to keep his eyes open due to the light's intensity. After the individual presses the second button the series of increasing light intensity flashes are stopped (this corresponds to the "Stop! Signal" and to the "End of assessment round" in figure 4). The last two levels of light intensity, i.e. the bearable one (i.e. light intensity level T1) just before the unbearable one (i.e. light intensity level T2) are averaged (i.e. (T1+T2)/2). Therefore the averaged light intensity is calculated from the last bearable discomfort and the first unbearable discomfort feelings (this last one corresponding to the "unbearable discomfort light intensity collected" of figure 4).

In another embodiment a single button is implemented and the individual is instructed to press the button when he feels for the first time the light's intensity uncomfortable (i.e. bearable discomfort) and press the button a second time when he feels for the first time the light's intensity unbearable (i.e. unbearable discomfort). In such a case, the first button pressing acts as the first button and the second button pressing acts as the second button in the above-described operations. Also, in such a case, the averaged light intensity (i.e. (T1+T2)/2) is calculated from the first bearable discomfort (i.e. start of discomfort, light intensity level T1) and the first unbearable discomfort (i.e. light intensity level T2) feelings. In other words, T1 is the first level of light intensity for which the individual or subject starts to fell discomfort and T2 is the first level of light intensity that is very uncomfortable such as to be unbearable.

This process is repeated/iterated four times for each individual to ensure a reliable estimation of the level of averaged light intensity at which the individual feels uncomfortable according to the above-mentioned subjective assessment, the four recorded averaged light intensities then being averaged together.

In the example of the Figure 4, the individual terminated the trial at the third light intensity due to an unbearable discomfort feeling, i.e. he pressed the second button (or pressed a second time the only one button) when being subjected to the third level of light intensity.

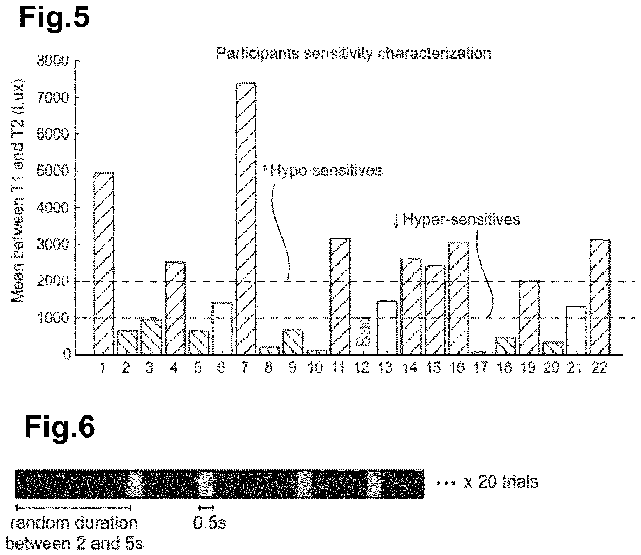

An example of the obtained levels of light intensities at which the individuals feel uncomfortable, i.e. subjective assessment, is represented Figure 5 in the case of a statistical study with 22 individuals.

From those obtained levels of light intensities of the individuals, it has been defined hyper-sensitive individuals who report discomfort for averaged light intensities between 1 lux and 1000 lux. Conversely, it has been defined hypo-sensitive individuals who report discomfort for averaged light intensities between 2000 lux and 12,000 lux. Individuals that start to feel uncomfortable between 1000 lux and 2000 lux are considered as "not extreme" and are not in the two above mentioned groups of hypo-sensitive and hyper-sensitive.
- In a subsequent phase (i.e. subsequent to the preliminary phase) of the statistical study, a study of the cortex activities of the individuals is done. It involves EEG recording sessions in which the cortex activity of each individual is recorded as a recorded cortical activity signal comprising EEG data, using an Electroencephalogram, i.e. EEG.

The EEG, a non-invasive technique, involves placing cranial electrodes on the scalp to measure the cortex's electrical impulses. In this particular study, the emphasis is on the immediate response of the cortex within the first 350 ms and preferably within the 300 ms from the moment the individual is starting to be exposed to a very dim light stimulus, preferably to a light intensity of 45 lux.

The rationale behind this is that an individual's instantaneous response can give valuable insights into their sensitivity to light, even before they consciously recognize or articulate it.

During the recording of the cortical activity, the individual (or the subject) is sitting in the dark in front of a light dome and maintains eye(s) fixation on the centre of the dome and remains as motionless as possible, with her/his head comfortably resting on a chinrest. When the individual (or the subject) receives the given light intensity, preferably 45 lux, the dome emits this faint light during preferably 300ms, that is also the duration of the EEG recording. Outside the periods the individual (or the subject) receives the given light intensity, no light is emitted by the dome and the subject is in the darkness/blackout during random durations ranging from 2 to 5 seconds as represented figure 6. This randomization of time intervals serves the purpose of preventing adaptation of the photoreceptors and the cortex's predictive mechanisms. Introducing variability in the dark periods minimizes any anticipatory responses and ensure more reliable measurements of cortex activity.

A single trial lasts on average less than 4 seconds and the entire procedure lasts 1 minute and 16 seconds. It is preferable to iterate the trial for each individual and to average the recordings. Preferably, for an individual (or the subject), the trial is iterated twenty times as represented figure 6 to give an optimal signal to noise ratio.
- Next a data Cleaning Phase is implemented.

Once the recorded cortical activity signal is obtained, it undergoes a stringent cleaning process to obtain a useful signal. Indeed, the recorded cortical activity signal data is often riddled with artifacts, i.e. unwanted signals that are not related to cortical activity. These artifacts can arise from various sources, such as eye movements, muscle activity, or external interference. It is preferable to remove these artifacts to ensure that the subsequent analysis is based purely on cortical responses and not confounded by extraneous noise. Various techniques, both manual and algorithmic, may be employed to sift through the data and remove these artifacts, leaving behind a useful signal comprising a clean dataset ready for the next phase of feature extraction.

More precisely, the EEG signal, i.e. the recorded cortical activity signal, undergo filtering with a high-pass filter at 1 Hz and a low-pass filter at 40 Hz. In addition, recordings/channels that contains artifacts are identified and filled in through interpolation. For that purpose of interpolation, it is necessary to extract statistically independent components from the EEG signals and therefore independent component analysis is applied. Only the most plausible independent components associated with cortex activity, with a probability (P) greater than or equal to 50%, are selected and used to reconstruct the signal in the electrode space, i.e. with correlations between multiple channels.

For that purpose, the time-series data are segmented into epochs, where each epoch begins 1 second before the stimulus onset and ends 2 seconds after the stimulus offset. This reconstruction through interpolation allows for subsequent analyses.

In all cases, the signal that is used for the assessment is the one measured and recorded between 0 and 350 ms, preferably between 0 and 300 ms from the onset of the light stimulus.
- Next a features extraction phase is implemented.

In this features extraction phase, distinct and meaningful features are extracted from the useful signals.

These features are essentially the specific cortical signature that is indicative of a sensitivity to light in the useful signal, and the predefined threshold parameter of the specific cortical signature that allow to differentiate between the two classes of individuals: individuals that are hyper-sensitives and individuals that are hypo-sensitives to the light.

It has been determined that the specific cortical signature is the evolution over time of an amplitude of the cortical activity signal of the cortex in the first 350 ms starting from the moment the at least one eye of the subject starts receiving the given light intensity. Said evolution over time of the amplitude of the cortical activity signal, in practical of the useful signal, which is in the time-domain, is indicative of an individual's sensitivity to light. The specific cortical signature which is in in the first 350 ms starting from stimulus onset, i.e. from the start of the given light intensity supplied to the individual (or subject), is an early response of the brain to the light stimulation.

The predefined threshold parameter is a statistical value or a set of statistical values descriptive of the evolution over time of the amplitude of the cortical activity signal and comprising at least one value from mean of the amplitude, standard deviation of the amplitude, variance of the amplitude, root mean square of the amplitude, minimum amplitude equal to 0µV, negative peak maximum absolute amplitude, positive peak maximum amplitude, a time latency of a determined peak (e.g. delay of the positive peak maximum amplitude from 0 ms, i.e. from the starts of the recording and of the subject receiving the given light intensity), peak-to-peak time separation of determined peaks (e.g. peak to peak time separation from the highest positive peak to the next one positive peak over time or peak to peak time separation from the highest positive peak to the highest positive peak of lower amplitude). Regarding those values and amplitude, the baseline of the signal is 0µV and a positive peak has a positive amplitude and a negative peak a negative amplitude.

Said threshold parameter defines/delimits a statistical boundary on the specific cortical signatures between two statistically defined sub-populations (or subject) from the defined population, a first sub-population of subjectively hypo-sensitive individuals (or subject) and a second sub-population of subjectively hyper-sensitive individuals;

Said threshold parameter correspond to a hyperplane obtained thanks to a machine learning classification process that preferably implements a Support Vector Machine (SVMs).

In this statistical study, the more the number of individuals, the more the accuracy of the classification results is enhanced.

Once, the preliminary threshold determination step is finished, the specific cortical signature having been identified and threshold parameter having been computed, it is possible to assess the sensitivity to light of subjects.

Of course, if the specific cortical signature and the threshold parameter are already or a *priori* known, there is no need to proceed with a preliminary threshold determination step.

The assessment is implemented through the steps a), b) and c) of the method of the invention. Said assessment is a classification operation in which an identified specific cortical signature of a useful signal from a subject is compared to a predefined threshold parameter.

The classification operation is done with machine learning algorithms or statistical methods in order to classify the new subject into either the hypo-sensitive or hyper-sensitive category/class.

It can be noted that if the use of a predefined threshold parameter is a simpler solution in terms of computational workload, it is also possible to implement processing means of the statistical study to classify the subject. However, this last solution is very demanding in terms of computational workload as it corresponds to a new statistical study on the population plus the subject.

Now, it is described more particularly the steps a), b) and c) of the method of assessment of sensitivity.

The step a) is an EEG recording session in which the neural activity from a subject is recorded thanks to the measuring and recording of the cortical activity signal of the cortex of the head of the subject while he/she is subjected to the given light intensity of 45 lux. Preferably, a single occipital electrode is used. The durations of the emission of light and of the measuring and recording are the same and starting at the same instant of time. In practical, the trial lasts 1 minute and 16 seconds.

In the recording, the signal between 0 and 350 ms, preferably between 0 and 300 ms from the onset of the light stimulus is used for the assessment. The trial is iterated, still with a light intensity of 45 lux, to obtain a plurality of cortical activity signals (or useful signals) for the same subject and that are then aggregated and averaged for further use, notably to identify the specific cortical signature (i.e. an Event-Related Potential (ERP) ) and then for the assessment.

For the assessment, the specific cortical signature found in the useful signal is computed to extract its descriptive statistics and values, said descriptive statistics corresponding to the ones of the predefined threshold parameter in order to allow a comparison to classify the subject as hyper-sensitive or hypo-sensitive.

For that purpose it can be used machine learning algorithms, for example: logistic regression, Support Vector Machines (SVMs), and Neural Networks, among others. If a machine-learning model is used for the assessment, once the machine-learning model is trained, the subject is instantly classified if he/she is hyper-sensitive or hypo-sensitive to light.

## Claims

1. Method for objectively assessing a level of sensitivity to a light entering at least one eye of a subject, comprising steps of:
a) measuring and recording a cortical activity signal of a cortex of a head of the subject while the at least one eye of the subject receives a given light intensity during a given measuring and recording time,
b) processing the recorded cortical activity signal to obtain a useful signal and to identify, from the useful signal, a specific cortical signature indicative of a sensitivity to light,
c) assessing whether the specific cortical signature correlates with a hypo-sensitivity to light or a hyper-sensitivity to light state of the subject by comparing the specific cortical signature to a predefined threshold parameter of the specific cortical signature,
wherein the given light intensity is less or equal to 50 lux and that the given measuring and recording time is less than or equal to 350 ms.

2. The method according to claim 1, wherein the cortical activity signal which is measured and recorded in step a) comprises regional parts originating from a plurality of different regions of the cortex and wherein the useful signal which is used to identify the specific cortical signature in step b) is obtained from at least these regional parts of the cortical activity signal originating from different regions of the cortex.

3. The method according to claim 2, wherein the cortical activity signal which is measured and recorded in step a) comprises three regional parts originating respectively from the occipital and frontal and mid-line regions of the cortex and wherein the useful signal which is used to identify the specific cortical signature in step b) is obtained mainly from these three regional parts of the cortical activity signal.

4. Method according to claim 2 or 3, wherein the cortical activity signal is measured and recorded exclusively from the occipital and/or frontal regions of the cortex.

5. Method according to claim 4, wherein the cortical activity signal is measured and recorded from only one cranial electrode and a reference electrode.

6. Method according to any one of claims 1 to 5, wherein the specific cortical signature is the evolution over time of an amplitude of the cortical activity signal of the cortex in the first 350 ms starting from the moment the at least one eye of the subject starts receiving the given light intensity.

7. Method according to claim 6, wherein the predefined threshold parameter is a statistical value or a set of statistical values descriptive of the evolution over time of an amplitude of the cortical activity signal and comprising at least one value from mean of the amplitude, standard deviation of the amplitude, variance of the amplitude, root mean square of the amplitude, minimum amplitude equal to 0µV, negative peak maximum absolute amplitude, positive peak maximum amplitude, time latency of a determined peak, peak-to-peak time separation of determined peaks.

8. Method according to any one of claims 1 to 7, wherein the predefined threshold parameter of the specific cortical signature is determined in a preliminary threshold determination step comprising a statistical computation of data related to cortical activities under different levels of light intensities of a plurality of individuals from a population distinct from the subject whose level of sensitivity to light is to be assessed in relation to the feeling of each individual as to whether he feels comfortable or uncomfortable with the light.

9. Method according to claim 8, wherein the preliminary threshold determination step comprises the sub-step of:
- for each individual:
-- measuring and recording a plurality of cortical activity signals while the at least one eye of the individual receives each time a different level of light intensity during a measuring and recording time less than or equal to 350 ms and processing the recorded cortical activity signals to obtain useful signals,
-- for each of the levels of light intensities, recording the feeling of the individual as to whether he feels comfortable or uncomfortable, and
- for all the individuals:
-- comparing with each other the useful signals and feelings of each individual or of all the individuals of the population to statistical compute said predefined threshold parameter of the specific cortical signature for which a change occurs between comfortable feelings and uncomfortable feelings.

10. Method according to any one of claims 1 to 7, wherein the predefined threshold parameter of the specific cortical signature is determined in a preliminary threshold determination step comprising a statistical computation of data related to cortical activities under different levels of light intensities of the subject which level of sensitivity to light is to be assessed, in relation to the feeling of the subject as to whether he/she feels comfortable or uncomfortable with the light.

11. Method according to claim 10, wherein the preliminary threshold determination step comprises the sub-step of:
-- measuring and recording a plurality of cortical activity signals of said subject while the at least one eye of the subject receives each time a different level of light intensity during a measuring and recording time less than or equal to 350 ms and processing the recorded cortical activity signals to obtain useful signals,
-- for each of the light intensities, recording the feeling of the subject as to whether he/she feels comfortable or uncomfortable,
-- comparing with each other the useful signals and feelings of the subject to statistically compute said predefined threshold parameter of the specific cortical signature for which a change occurs between comfortable feelings and uncomfortable feelings.

12. Method according to any one of claims 1 to 11, further comprising a step of determining, based on the result of assessing step c), a parameter that is characteristic of a light filter to be provided to the subject in order to maintain or improve the visual comfort and/or visual acuity of the subject.

13. A device for objectively assessing a level of sensitivity to a light entering at least one eye of a subject and comprising a control unit configured to execute the steps of:
a) measuring and recording a cortical activity signal of a cortex of a head of the subject while the at least one eye of the subject receives a given light intensity during a given measuring and recording time, the given light intensity being less or equal to 50 lux and the given measuring and recording time is less than or equal to 350 ms,
b) processing the recorded cortical activity signal to obtain a useful signal and to identify, from the useful signal, a specific cortical signature indicative of a sensitivity to light,
c) assessing whether the specific cortical signature correlates with a hypo-sensitivity to light or a hyper-sensitivity to light state of the subject by comparing the specific cortical signature to a predefined threshold parameter of the specific cortical signature,
said device comprising at least one light source to provide the given light intensity during the given measuring and recording time.

14. An eyewear comprising:
- a device for objectively assessing a level of sensitivity to a light entering at least one eye of a subject and comprising at least one cranial electrode and a control unit configured to execute the steps of:
a) measuring and recording a cortical activity signal of a cortex of a head of the subject while the at least one eye of the subject receives a given light intensity during a given measuring and recording time, the given light intensity being less or equal to 50 lux and the given measuring and recording time is less than or equal to 350 ms,
b) processing the recorded cortical activity signal to obtain a useful signal and to identify, from the useful signal, a specific cortical signature indicative of a sensitivity to light,
c) assessing whether the specific cortical signature correlates with a hypo-sensitivity to light or a hyper-sensitivity to light state of the subject by comparing the specific cortical signature to a predefined threshold parameter of the specific cortical signature.

15. The eyewear of claim 14, comprising a light source to provide the subject with the given light intensity.
